Europäisches Patentamt

European Patent Office

Office européen des brevets

(19)

(11) EP 0 925 826 B1

(12) FASCICULE DE BREVET EUROPEEN

(45) Date de publication et mention
de la délivrance du brevet:
**27.10.2004 Bulletin 2004/44**

(51) Int Cl.7: **B01D 71/42**, B01D 67/00

(21) Numéro de dépôt: **98420239.0**

(22) Date de dépôt: **18.12.1998**

(54) **Utilisation d'un polymère neutre ou cationique pour prévenir l'activation de la phase contact du sang ou du plasma en contact avec une membrane semi-perméable**

Verwendung eines neutralen oder kationischen Polymers zur Verhinderung der Aktivierung der Blut- oder Plasmakontaktphase in Berührung mit einer teildurchlässigen Membran

Use of a neutral or cationic polymer for preventing the activation of blood or plasma contact phase in contact with a semipermeable membrane

(84) Etats contractants désignés:
**AT BE CH DE ES FR GB IT LI NL SE**

(30) Priorité: **24.12.1997 FR 9716732**

(43) Date de publication de la demande:
**30.06.1999 Bulletin 1999/26**

(73) Titulaire: **HOSPAL INDUSTRIE
F-69883 Meyzieu Cédex (FR)**

(72) Inventeurs:
• **Thomas, Michel
69360 Serezin-du-Rhone (FR)**
• **Valette, Pierre
69003 Lyon (FR)**

(74) Mandataire: **Sutto, Luca et al
Gambro Intellectual Property Department,
P.O. Box 98
41037 Mirandola (MO) (IT)**

(56) Documents cités:
**EP-A- 0 561 379          EP-A- 0 801 953**

• **DATABASE WPI Week 9627 Derwent
Publications Ltd., London, GB; AN 96-266673
XP002079254 & SU 1 343 596 A (LENGD
SANITARY HYGIENE MED INST,LENGD
TEXTILE LIGHT IND INST) , 10 novembre 1995**

**Description**

La présente invention a pour objet l'utilisation d'un polymère, neutre ou cationique, combiné avant stérilisation à une membrane semi-perméable à base de polyacrylonitrile porteur de charges négatives fixes, prévue dans un appareil pour le traitement du sang ou du plasma par circulation extracorporelle, pour prévenir l'activation de la phase contact du sang ou du plasma.

La présente invention a également pour objet un appareil pour le traitement du sang ou du plasma par circulation extracorporelle permettant de prévenir l'activation de la phase contact du sang ou du plasma, ainsi que des procédés de fabrication de cet appareil.

Des appareils pour le traitement du sang ou du plasma par circulation extracorporelle sont utilisés dans diverses applications médicales ou paramédicales telles que : traitement de l'insuffisance rénale par dialyse ou hémofiltration, plasmaphérèse et aphérèse à visée thérapeutique et non thérapeutique, oxygénation du sang, immunoépuration, etc.

L'activation de la phase contact du sang ou du plasma semble se produire notamment quand on utilise un appareil pour le traitement du sang ou du plasma par circulation extracorporelle, renfermant une membrane semi-perméable chargée négativement, sans que, en l'absence de facteurs perturbateurs, les patients en éprouvent le moindre inconfort. L'activation de la phase contact est décrite comme un phénomène biologique qui se produit chez les patients soumis à un traitement du sang (ou du plasma) par circulation extracorporelle, lorsque le sang entre en contact avec la surface chargée négativement de la membrane de certains appareils pour le traitement du sang et du plasma. Ce phénomène biologique aboutit à la génération de substances actives, la kallicréine et le facteur XIIa à partir de substances inactives, prékallicréine et facteur XII, la kallicréine ayant un effet catalytique sur la production du facteur XIIa et inversement. En outre, la kallicréine est à l'origine de la transformation d'une protéine du plasma, le kininogène de haut poids moléculaire, en une substance peptidique, la bradykinine.

Lorsque l'activation de la phase contact se produit simultanément à certains facteurs perturbateurs comme, par exemple :

- la présence, dans le sang à traiter, de médicaments pour combattre l'hypertension artérielle par inhibition du mécanisme naturel de la vasoconstriction, génériquement désignés par le vocable d'inhibiteurs d'enzyme de conversion ou IEC. Ces IEC sont également utilisés pour d'autres applications thérapeutiques, notamment pour traiter certaines formes d'insuffisance cardiaque,

- la dilution du sang entrant dans l'appareil rempli de solution saline et l'abaissement concomitant du pH du sang,

l'activation de la phase contact semble à l'origine de réactions indésirables dites anaphylactoïdes.

Ces réactions anaphylactoïdes se manifestent quelques minutes après le début du traitement par divers symptômes, parmi lesquels la sensation de chaleur généralisée, l'engourdissement des doigts, des lèvres ou de la langue, le halètement, la nausée, l'oedème laryngé.

Des réactions anaphylactoïdes ont été observées notamment chez les insuffisants rénaux traités par hémodialyse, hémofiltration ou hémodiafiltration au moyen d'un appareil pour le traitement du sang sous la forme d'un échangeur à membrane ou dialyseur.

Des réactions anaphylactoïdes ont été observées avec des échangeurs ayant des membranes de différentes compositions chimiques, tantôt lors d'une première utilisation, tantôt après plusieurs utilisations lorsque les échangeurs, au lieu d'être jetés après un usage unique, sont réutilisés de multiples fois et sont recyclés après chaque usage. Comme exemple d'échangeurs dont une première utilisation s'est accompagnée d'une réaction indésirable, on peut citer les dialyseurs ayant une membrane à base de polyméthylméthacrylate et de polyacrylonitrile. Des réactions associées à la réutilisation des dialyseurs à membrane à base d'acétate de cellulose et de polysulfone ont été également bien documentées (voir l'article intitulé «Anaphylactoid reactions associated with reuse of hollow-fiber hemodialyzers and ACE inhibitors» dans «Kidney International», vol. 42 (1992), pp. 1232-1237).

Les réactions anaphylactoïdes sont imputées à une concentration excessive de bradykinine dans le sang ou le plasma.

Afin d'éviter la génération de bradykinine à une concentration supérieure à 4000 pg/ml, le brevet européen n° 0561379 recommande de ne mettre en contact avec le sang ou le plasma que des membranes semi-perméables présentant une densité de charges limitées en surface, à savoir une charge électrique globale en surface supérieure ou égale à - 30 $\mu$eq/g de membrane, cette charge électrique étant mesurée par une méthode choisie dans le groupe constitué de la méthode d'adsorption de colorants, de la méthode de coupure de sels, de la méthode de titrage jusqu'à neutralisation et de la méthode à l'iodure.

Mais, de la description de l'invention revendiquée dans le brevet européen n°0561379, en particulier des méthodes de mesure proposées, il ressort que la charge électrique en surface correspond en fait à la capacité ionique globale des membranes semi-perméables. Dès lors, ce brevet européen ne concerne que les membranes semi-perméables ayant une capacité ionique globale supérieure ou égale à - 30 µeq/g de membrane, mais pas les membranes semi-perméables ayant une capacité ionique globale très inférieure à -30 µeq/g de membrane comme, par exemple, la membrane fabriquée par la société HOSPAL à partir d'un copolymère d'acrylonitrile et de méthallylsulfonate de sodium connu sous le nom commercial AN69.

Or, il est souhaitable de ne pas modifier la capacité ionique des membranes car cette capacité favorise l'adsorption et/ou le transfert de : certaines protéines telles que la β2-microglobuline, les médiateurs de l'inflammation et les facteurs du complément; les lipides. Cela est particulièrement vrai pour les membranes ayant une structure homogène, symétrique.

Par ailleurs, la demanderesse a observé, sur plusieurs exemples, qu'une membrane possédant une capacité ionique globale supérieure à -30 µeq/g de membrane, peut conduire à une activation de la phase contact tandis que, à l'inverse, une membrane de capacité ionique globale nettement inférieure à -30 µeq/g de membrane peut ne pas conduire à une activation de la phase contact.

Le document EP 0 801 953, déposé au nom du demandeur, concerne une membrane normalement chargée négativement. La réaction du sang avec une telle membrane, appelée activation de la phase contact, n'est pas souhaitable pour le patient et le problème posé par ce document est d'éviter une telle réaction. L'invention concerne un traitement de surface grâce auquel l'électronégativité de la surface est réduite pour prévenir l'activation de la phase contact. Il est utilisé une action biologique selon laquelle un agent cationique et antiprotéase (appelé inhibiteur de protéase) est incorporé dans au moins une partie de la membrane, cet agent étant adsorbé dans la membrane et/ou lié par interaction ionique avec les sites électronégatifs de la membrane. L'action biologique est utilisée avant ou après la formation de la membrane, et avant la stérilisation de l'appareil.

Pour « contrer » l'activation de phase contact, l'inhibiteur de protéase agit à un certain moment de cette réaction : en effet, il n'empêche pas l'activation de phase contact de débuter, en revanche il inhibe des produits intermédiaires de ladite activation, appelés kallicréine.

Compte tenu de ce qui précède, à cette date, une solution satisfaisante, à la fois sur le plan médical et le plan économique, permettant de prévenir l'activation de la phase contact du sang ou du plasma venant en contact d'une membrane semi-perméable chargée négativement, ne semble pas connue.

Un but de l'invention est donc de résoudre le problème précité avec un appareil pour le traitement du sang ou du plasma par circulation extracorporelle comprenant une membrane semi-perméable à base de polyacrylonitrile porteur de charges négatives fixes, qui possède deux caractéristiques considérées comme antinomiques jusqu'à présent, à savoir :

- une capacité ionique globale négative, correspondant à un excès de charges négatives, qui tout à la fois participe à la biocompatibilité de la membrane et est un facteur de déclenchement de l'activation de la phase contact,

- et une aptitude à ne pas produire une activation de la phase contact dans des conditions normales d'une première utilisation.

Un autre but de l'invention est de résoudre le problème précité avec un appareil stérilisé pour le traitement du sang ou du plasma par circulation extracorporelle qui présente une aptitude à ne pas produire une activation de la phase contact dans des conditions normales d'une première utilisation et qui est stable au stockage. Enfin, un autre but de l'invention est un appareil stérilisé pour le traitement du sang ou du plasma par circulation extracorporelle qui présente une aptitude à ne pas produire une activation de la phase contact dans des conditions normales d'une première utilisation et qui est prêt à l'emploi, c'est-à-dire qui ne requière pas de manipulation spéciale de la part de l'utilisateur de l'appareil, telle qu'une manipulation spéciale aux fins de prévenir les effets indésirables de l'activation de la phase contact.

A cet effet, l'invention propose l'utilisation d'un polymère neutre ou cationique pour prévenir l'activation de la phase contact du sang ou du plasma venant au contact d'une membrane semi-perméable à base de polyacrylonitrile porteur de charges négatives fixes, prévue dans un appareil pour le traitement du sang ou du plasma, par circulation extracorporelle, selon laquelle :

(1) le polymère, neutre ou cationique, est combiné à cette membrane semi-perméable, avant ou après formation de la membrane, et avant stérilisation de la membrane ;
(2) la capacité ionique globale de la membrane semi-perméable, à base de polyacrylonitrile porteur de charges négatives fixes et renfermant un polymère neutre ou cationique, est inférieure à - 30 µeq/g de polymère gouflé;
(3) la capacité ionique globale de la membrane semi-perméable, à base de polyacrylonitrile porteur de charges négatives fixes et renfermant un polymère neutre ou cationique, est, en valeur absolue, au plus de 10% inférieure à la capacité ionique globale de la même membrane semi-perméable, exempte de polymère neutre ou cationique ;

de préférence, elle est, en valeur absolue, au , plus de 1% inférieure à la capacité ionique globale de la même membrane semi-perméable, exempte de polymère neutre ou cationique;

(4) la membrane semi-perméable renfermant un polymère neutre ou cationique vérifie, avant stérilisation, l'une ou l'autre des deux caractéristiques électriques suivantes :

- l'indice électrocinétique «I» qui est égal à $Log_{10}(|Z|/R)$ c'est-à-dire au logarithme, en base 10, du rapport $|Z|/R$ du potentiel Zêta «Z», en valeur absolue, exprimé en microvolt (ou µV), de la surface de la membrane destinée à venir au contact du sang ou du plasma, sur la résistivité électrique «R» de la membrane exprimée en ohm.centimètre (ou $\Omega$.cm), est inférieur ou égal à 0,8, ou

- le potentiel Zêta « Z » est positif et est compris entre 0 et + 15 mV, (bornes non incluses).

De préférence, l'indice électrocinétique «I» est inférieur ou égal à 0,7, encore mieux, inférieur ou égal à 0,6.

De préférence, le potentiel Zêta, lorsqu'il est positif, est inférieur strictement à 15 mV et supérieur ou égal à 1 mV. Encore mieux, le potentiel Zêta est supérieur ou égal à 6 mV et inférieur strictement à 15 mV.

Pour obtenir l'indice électrocinétique «I» :

- le potentiel Zêta «Z» est calculé à partir de la mesure du potentiel «E», exprimé en volt, créé par l'écoulement d'un électrolyte (NaCl $10^{-2}$M), par exemple à l'intérieur d'un faisceau de fibres creuses. Ce potentiel «E», mesuré entre deux électrodes Ag/AgCl connectées à un voltmètre à impédance élevée (KEITHLEY 617), est relié au potentiel Zêta «Z», exprimé en volt, par la loi d'Helmholtz Smoluchowski :

$$Z = \frac{4\,\pi\,v\,\lambda\,E}{\varepsilon P}$$

où :

. P est la pression hydrostatique provoquant l'écoulement de l'électrolyte, en millimètre de mercure (mmHg) (le rapport E/p est nommé potentiel d'écoulement),
. v est la viscosité dynamique de l'électrolyte, en Pascal, .
. $\lambda$ est la conductivité électrique réelle du système en équilibre avec l'électrolyte (obtenue par une mesure de résistance avec NaCl $10^{-2}$M) et est exprimée en Siemens/m, .
. $\varepsilon$ est la constante diélectrique de l'électrolyte ou la permittivité.

- et la résistivité électrique «R», exprimée en $\Omega$.cm, est déduite d'une mesure de résistance électrique de la membrane semi-perméable en équilibre avec un électrolyte (NaCl $5.10^{-5}$M), en utilisant un pont de WHEATSTONE qui fonctionne avec un courant alternatif (générateur WAWETEK, Modèle 19, Fréquence 10Hz).

La détermination expérimentale des caractéristiques électriques de la membrane semi-perméable renfermant un polymère neutre ou cationique doit être effectuée avant stérilisation de la membrane dans le cas d'une stérilisation par irradiation. Sinon, les valeurs obtenues sont aberrantes et ne peuvent corrélées directement avec la quantité de polymère neutre ou cationique à utiliser. Dans les autres modes de stérilisation, moins énergétique comme la stérilisation par l'oxyde d'éthylène, cette détermination expérimentale peut être effectuée avant ou après stérilisation.

Dans le cadre de l'invention, on estime que l'activation de la phase contact est effective dès lors que la concentration maximale de kallicréines (KK) produites pendant les 10 premières minutes de contact avec le sang ou le plasma excède 10 unités de kallicréines (KK) par litre de sang ou de plasma (10 UKK/l), compte tenu de la sensibilité du test chromogénique utilisé.

Par «membrane semi-perméable», on entend une membrane plane ou un faisceau de fibres creuses. Dès lors, l'appareil pour le traitement du sang ou du plasma par circulation extracorporelle comprend, en général, deux compartiments séparés par la membrane semi-perméable.

Par «membrane semi-perméable à base de polyacrylonitrile porteur de charges négatives fixes», on entend une membrane semi-perméable constituée de polyacrylonitrile auquel des groupes fonctionnels anioniques sont liés par des liaisons covalentes, ce polyacrylonitrile chargé négativement n'étant pas hydrosoluble.

Par «polymère neutre ou cationique combiné (ou incorporé) à la membrane semi-perméable», on entend que ce polymère est introduit :

- soit dans la masse du polyacrylonitrile chargé négativement (ce procédé convient plus particulièrement au polymère neutre),

-   soit à la surface de la membrane semi-perméable, par exemple par mise en contact de la membrane avec une solution contenant le polymère (ce procédé convient plus particulièrement au polymère cationique) ou bien par pulvérisation d'une solution contenant le polymère.

En outre, les conditions opératoires pour réaliser cette combinaison (ou incorporation) sont prévues pour favoriser la présence d'une partie au moins du polymère neutre ou cationique à la surface de la membrane semi-perméable (par exemple, par migration favorisée du polymère neutre ou liaison ionique du polymère cationique).

Par «polymère gonflé», on entend ici la membrane, hydratée à la teneur correspondant à l'utilisation clinique.

De façon surprenante, il a été trouvé qu'il est possible de prévenir l'activation de la phase contact, qui peut survenir épisodiquement lors de l'utilisation d'un appareil pour le traitement du sang ou du plasma par circulation extracorporelle, au moyen d'une membrane semi-perméable à base de polyacrylonitrile porteur de charges négatives fixes, en modifiant les caractéristiques électriques de la membrane, de façon que l'une ou l'autre des deux conditions suivantes soient remplies avant stérilisation :

-   l'indice électrocinétique «I», qui est égal à $Log_{10} |Z|/R$ est inférieur ou égal à 0,8,
-   ou, le potentiel Zêta « Z » est positif et varie entre 0 et + 15 mV (bornes non incluses),

tout en maintenant substantiellement la capacité ionique globale de la membrane, la modification de ces caractéristiques électriques étant obtenu en combinant à cette membrane un polymère neutre ou cationique en quantité appropriée, cette combinaison étant effectuée :

-   premièrement avant stérilisation,
-   deuxièmement, avant ou après formation de cette membrane.

La prévention de l'activation de la phase contact a, en particulier, été obtenue avec des membranes semi-perméables, à base de polyacrylonitrile fortement chargées négativement, atteignant des capacités ioniques globales inférieures ou égales à - 100 µeq/g de polymère gonflé.

De façon surprenante également, la stérilisation de l'appareil n'a pas d'influence sur son aptitude à prévenir l'activation de la phase contact.

Dans les conditions normales d'utilisation de l'appareil selon l'invention, les qualités connues de la membrane semi-perméable sont conservées intactes quand cette même membrane renferme un polymère neutre ou cationique : par exemple, pour une membrane d'hémodialyse/hémofiltration, l'hémocompatibilité, les performances en terme de transferts diffusifs et convectifs, la capacité d'adsorption de substances indésirables, etc, sont conservées intactes.

De façon intéressante, il n'est pas constaté d'adsorption d'héparine, sur une membrane semi-perméable, à base de polyacrylonitrile porteur de charges négatives fixes, modifiée conformément à la présente invention, dans le cas où la stérilisation est effectuée par irradiation. En outre, l'adsorption d'héparine n'est pas rédhibitoire en soi et peut présenter des avantages pour certains types de traitement du sang, la membrane étant en effet, dans ce cas, moins thrombogène.

Selon l'invention, la membrane semi-perméable à base de polyacrylonitrile chargé négativement, avant combinaison avec un polymère neutre ou cationique, présente une densité de charges électronégatives en surface correspondant à des charges négatives en excès détectables, en particulier par les mesures du potentiel électrocinétique (potentiel Zêta). Le polymère neutre ou cationique permet de masquer, pour partie au moins, les charges électronégatives présentes à la surface de la membrane. Dans le cas d'un polymère cationique, le masquage de charges négatives de la membrane est réalisé notamment par liaison ionique.

De préférence, le polymère neutre est hydrosoluble à température ambiante (de l'ordre de 20° C).

De préférence, le polymère cationique est hydrosoluble à température ambiante (de l'ordre de 20° C). Toutefois, un polymère cationique soluble dans un solvant organique, tels que les alcools, peut convenir à l'invention.

Le polymère neutre ou cationique doit pouvoir supporter une stérilisation énergétique, du type irradiation gamma. En d'autres termes, au moins une partie du polymère doit rester intacte et pouvoir masquer, de la façon souhaitée, une partie des charges électronégatives en surface de la membrane. Par ailleurs, le polymère fixé à la membrane et irradié ne doit pas devenir toxique.

La masse moléculaire moyenne en poids du polymère est au moins égale à 10000 Daltons.

Avantageusement, la masse moléculaire moyenne en poids du polymère neutre est supérieure à 40.000 Daltons, de préférence supérieure à 100.000 Daltons et la masse moléculaire moyenne du polymère cationique est supérieure à 25.000 Daltons, de préférence supérieure à 100.000 Daltons. Ces masses moléculaires sont mesurées par une méthode de diffusion de lumière. A titre de polymère convenant à la réalisation de la présente invention, on peut citer, à titre de polymère neutre, les polyvinylpyrrolidones (PVP) et les polyéthylèneglycols (PEG) de différentes masses moléculaires ; à titre de polymère cationique, des polymères hydrophiles cationiques capables de s'adsorber sur une membrane semi-perméable présentant une densité de charges électronégatives en surface tels que les polyamines, par exemple les polyéthylèneimines (PEI), les diéthylaminoéthyles dextran ou DEAE dextran, et les polymères et copolymères contenant un ou plusieurs groupes ammoniums quaternaires.

Selon une variante d'exécution préférée de l'invention, le polymère est cationique. De préférence encore, il est choisi parmi les polyéthylèneimines (PEI).

La quantité de polymère neutre ou cationique à combiner à la membrane est fonction des caractéristiques électriques (Z, R, I) visées et est variable selon la nature chimique du polymère, mais n'excède pas 10% de la masse de polyacrylonitrile. Cette quantité est généralement au plus égale à 2% de la masse de polyacrylonitrile constituant la membrane dans le cas du polymère neutre.

La quantité de polymère cationique à combiner à la membrane est comprise de préférence entre environ 1 et environ 10 mg par $m^2$ de membrane destinée à être au contact du sang ou du plasma (cette quantité est très inférieure à 1% de la masse de polyacrylonitrile constituant la membrane).

L'invention est particulièrement bien adaptée aux membranes semi-perméables à base de polyacrylonitrile porteur de charges négatives fixes lui conférant une capacité ionique globale élevée en valeur absolue, même après incorporation du polymère neutre ou cationique.

Ainsi, l'invention est particulièrement bien adaptée aux membranes semi-perméables, à base de polyacrylonitrile porteur de charges négatives fixes et renfermant un polymère neutre ou cationique, qui présentent une capacité ionique globale inférieure à -30 µeq par g de polymère gonflé (i.e. la membrane), de préférence inférieure à -50 µeq par g de polymère gonflé, telle que mesurée par la méthode classique d'échanges d'ions : à titre de référence, le polymère électronégatif utilisé pour réaliser la membrane en AN69 présente une capacité ionique globale ou densité de charges négatives égale à environ - 180 µeq par g de polymère gonflé.

Avantageusement, la membrane semi-perméable est une membrane plane ou un faisceau de fibres creuses à base d'un homopolymère ou un copolymère d'acrylonitrile et associé à un polymère neutre ou cationique. A titre d'exemples de copolymères d'acrylonitrile, on peut citer :

(1) un copolymère de l'acrylonitrile et d'au moins un monomère anionique ou anionisable, renfermant, le cas échéant, des unités provenant d'au moins un autre monomère à insaturation oléfinique capable d'être copolymérisé avec l'acrylonitrile, ou

(2) un copolymère de l'acrylonitrile et d'au moins un monomère non ionique et non ionisable, renfermant, le cas échéant, des unités provenant d'au moins un autre monomère à insaturation oléfinique capable d'être copolymérisé avec l'acrylonitrile

Certains de ces polyacrylonitriles, ainsi que les divers monomères susceptibles d'être retenus comme matières premières et leur fabrication, sont plus amplement décrits dans le brevet américain n° 4,545,910 redélivré sous le n° Re.34239.

Parmi ces polyacrylonitriles, ceux avec lesquels l'invention est particulièrement bien adaptée sont définis sous (1) ci-avant. En particulier, l'invention convient particulièrement bien à ceux pour lesquels le comonomère anionique ou anionisable est oléfiniquement insaturé et porteur de groupements anioniques choisis parmi les groupes sulfonate, carboxyle, phosphate, phosphonate et sulfate, et plus particulièrement encore, quand ce comonomère est le méthallylsulfonate de sodium.

La nature précise du contre-ion des groupements anioniques n'est pas essentielle au bon fonctionnement de l'invention.

L'invention a également pour objet des procédés de fabrication d'un appareil pour le traitement du sang ou du plasma par circulation extracorporelle permettant de prévenir l'activation de la phase contact et comprenant une membrane semi-perméable sous la forme d'une membrane plane ou d'un faisceau de fibres creuses, à base de polyacrylonitrile porteur de charges négatives fixes et combiné à un polymère neutre ou cationique.

Un premier procédé de fabrication comprend les étapes de :

. préparer une solution constituée de :

- au moins un polyacrylonitrile porteur de charges négatives fixes ;

- au moins un polymère neutre dans une quantité ajustée pour obtenir les quatre caractéristiques (1)-(4) précitées,

- au moins un solvant dudit polyacrylonitrile et du polymère neutre,

- éventuellement, au moins un non solvant du polyacrylonitrile,

- extruder cette solution pour former une fibre creuse ou une membrane plane ;
- simultanément dans le cas de la préparation d'une fibre creuse ou après l'extrusion dans le cas de la formation d'une membrane plane, solidifier la membrane obtenue par un procédé d'inversion de phase par contact partiel ou total du produit extrudé avec un fluide, liquide ou gazeux, et chimiquement inerte vis-à-vis desdits polymères ;
- laver la membrane plane ou la fibre creuse obtenue ;
- éventuellement, glycériner la membrane plane ou la fibre creuse ;
- préparer une membrane semi-perméable à partir de la membrane plane ou conformer un faisceau de fibres creuses à partir de la fibre creuse ;
- monter la membrane plane ou le faisceau de fibres creuses dans un boîtier et, le cas échéant, fixer des embouts au boîtier ;
- stériliser l'appareil médical obtenu.

Dans le cas où le polymère est cationique, on peut l'associer à la membrane semi-perméable, après l'étape d'extrusion permettant d'obtenir une fibre creuse ou une membrane plane, selon un second procédé comprenant les étapes de :

(a) préparer une membrane plane ou une fibre creuse, éventuellement glycérinée, par un procédé conventionnel à partir d'une solution de polyacrylonitrile porteur de charges négatives ;
(b) assembler, de façon classique, les divers composants de l'appareil, en particulier monter la membrane semi-perméable ou un faisceau de fibres creuses dans un boîtier et réaliser les embouts de ce boîtier ;
(c) simultanément ou successivement, éventuellement déglycériner la membrane semi-perméable et préparer une solution contenant le polymère cationique à l'état dissous et porter cette solution au contact de la surface de la membrane semi-perméable destinée à être mise en contact avec le sang, l'étape (c) pouvant être réalisée avant ou après l'étape (b), la quantité de polymère cationique étant ajustée pour obtenir les quatre caractéristiques (1)-(4) précitées ;
(d) dans le cas où l'étape (c) précitée a été réalisée postérieurement à l'étape (b), purger l'appareil de la solution contenant le polymère cationique ;
(e) éventuellement, rincer la membrane semi-perméable pour éliminer l'excédent de polymère cationique non fixé et, éventuellement, reglycériner la membrane semi-perméable ;
(f) stériliser l'appareil médical.

Avantageusement, le polymère cationique est hydrosoluble et la solution dans laquelle ce polymère est dissout est aqueuse.

Dans le cas d'une membrane plane semi-perméable, on peut lui associer un polymère neutre ou cationique, de préférence cationique, en utilisant un procédé de pulvérisation comprenant les étapes suivantes :

(a) préparer une membrane plane, éventuellement glycérinée, à partir d'une solution de polyacrylonitrile porteur de charges négatives;
(b) simultanément ou successivement, éventuellement déglycériner la membrane semi-perméable et préparer une solution contenant le polymère cationique ou neutre à l'état dissous et pulvériser cette solution sur la surface de la membrane semi-perméable destinée à être mise en contact avec le sang, la quantité de polymère cationique ou neutre étant ajustée pour obtenir les quatre caractéristiques (1)-(4) énoncées ci-dessus ;
(c) assembler les divers composants de l'appareil, en particulier monter la membrane semi-perméable dans un boîtier et réaliser les embouts de ce boîtier;
(d) éventuellement, rincer la membrane semi-perméable pour éliminer l'excédent de polymère cationique non fixé et, éventuellement, reglycériner la membrane semi-perméable ;
(f) stériliser l'appareil médical.

En outre, dans le cadre des procédés de fabrication précités d'un appareil pour le traitement du sang ou du plasma par circulation extracorporelle permettant de prévenir l'activation de la phase contact et comprenant une membrane semi-perméable sous la forme d'une membrane plane ou d'un faisceau de fibres creuses, à base de polyacrylonitrile porteur de charges négatives fixes et combiné à un polymère neutre ou cationique, la quantité de polymère, qu'il soit neutre ou cationique, est ajustée pour remplir les conditions suivantes :

- la capacité ionique globale de la membrane semi-perméable, à base de polyacrylonitrile porteur de charges négatives fixes et renfermant un polymère (neutre ou cationique) est inférieure à - 30 $\mu$eq/g de polymère gouflé;

- la capacité ionique globale de la membrane semi-perméable, à base de polyacrylonitrile porteur de charges négatives fixes et renfermant un polymère (neutre ou cationique) est, en valeur absolue, au plus de 10% inférieure à la capacité ionique globale de la même membrane semi-perméable, exempte de polymère (neutre ou cationique) ; de préférence, elle est, en valeur absolue, au plus de 1% inférieure à la capacité ionique globale de la même membrane semi-perméable, exempte de polymère ;

- les caractéristiques électriques de la membrane semi-perméable renfermant un polymère (neutre ou cationique) remplissent l'une ou l'autre des deux conditions qui suivent :

  . l'indice électrocinétique «I» est égal à $\text{Log}_{10}(|Z|/R)$ c'est-à-dire au logarithme, en base 10, du rapport $|Z|/R$ du potentiel Zêta «Z», en valeur absolue, exprimé en microvolt (ou $\mu V$), de la surface de la membrane destinée à venir au contact du sang ou du plasma, sur la résistivité électrique «R» de la membrane exprimée en ohm. centimètre (ou $\Omega.cm$), est inférieur ou égal à 0,8,

  . ou le potentiel Zêta « Z » est positif et est compris entre 0 et + 15 mV (bornes non incluses).

De préférence, l'indice électrocinétique «I» est inférieur ou égal à 0,7, encore mieux, inférieur ou égal à 0,6.

De préférence, le potentiel Zêta, lorsqu'il est positif, est supérieur ou égal à 1 mV et inférieur strictement à 15 mV.

D'autres conditions opératoires de préparation de la membrane semi-perméable pourront être trouvées dans le brevet US4749619 (procédé par gélification) ou dans le brevet US4056467 (procédé par coagulation).

Selon le cas, la technique de stérilisation qui sera employée, sans effet significatif sur la liaison entre le polymère neutre ou cationique et la membrane semi-perméable à base de polyacrylonitrile, pourra être la stérilisation par irradiation, en particulier par irradiation gamma, ou la stérilisation à l'oxyde d'éthylène.

Les procédés précités de fabrication d'un appareil pour le traitement du sang ou du plasma par circulation extra-corporelle présentent un avantage majeur : les appareils obtenus ne requièrent pas de manipulation particulière de la part de l'utilisateur, en particulier pendant les phases de rinçage et d'amorçage des appareils, et la mise en oeuvre des appareils par l'utilisateur est exactement identique à celle de tout appareil du même type.

L'invention a enfin pour objet un appareil pour le traitement du sang ou du plasma par circulation extracorporelle, stérilisé et prêt à l'emploi, permettant de prévenir l'activation de la phase contact et comprenant une membrane semi-perméable, sous la forme d'une membrane plane ou d'un faisceau de fibres creuses, à base de polyacrylonitrile porteur de charges négatives fixes, caractérisé en ce que avant ou après formation de la membrane semi-perméable, et avant stérilisation, au moins un polymère neutre ou cationique est incorporé à la membrane semi-perméable, en une quantité appropriée de sorte que :

- la capacité ionique globale de la membrane semi-perméable à base de polyacrylonitrile porteur de charges négatives fixes et renfermant un polymère neutre ou cationique, est inférieure à - 30 $\mu$eq/g de polymère gouflé;

- la capacité ionique globale de la membrane semi-perméable à base de polyacrylonitrile porteur de charges négatives fixes et renfermant un polymère neutre ou cationique, est, en valeur absolue, au plus de 10% inférieure à la capacité ionique globale de la même membrane semi-perméable, exempte de polymère neutre ou cationique ;

- la membrane semi-perméable à base de polyacrylonitrile porteur de charges négatives fixes et renfermant un polymère neutre ou cationique vérifie l'une ou l'autre des caractéristiques électriques qui suivent, avant stérilisation :

  * l'indice électrocinétique «I» de la membrane, renfermant un polymère neutre ou cationique est inférieur ou égal à 0,8, «I» étant égal au logarithme, en base 10, du rapport $|Z|/R$, où «Z» est le potentiel Zêta, en valeur absolue, exprimé en microvolt, de la surface de la membrane destinée à venir au contact du sang ou du plasma, et où «R» est la résistivité électrique de la membrane exprimée en ohm.centimètre,

  * ou, le potentiel Zêta est positif et varie entre 0 et + 15 mV (bornes non incluses).

De préférence, l'indice électrocinétique «I» est inférieur ou égal à 0,7, encore mieux, inférieur ou égal à 0,6.

De préférence, le potentiel Zêta, lorsqu'il est positif, est supérieur ou égal à 1 mV et inférieur strictement à 15 mV.

## EXEMPLES ILLUSTRATIFS ET NON LIMITATIFS DE L'INVENTION

Exemple 1

Un minidialyseur comprenant 170 fibres creuses en AN69 (membrane constituée d'un copolymère d'acrylonitrile et de métallylsulfonate de sodium) a été assemblé. Le compartiment sang, ou compartiment interne, est délimité par l'intérieur des fibres et deux embouts munis chacun d'une tubulure d'accès, fixés aux extrémités du boîtier du minidialyseur.

Chaque fibre a les dimensions suivantes :

- diamètre interne : 240 μm,
- épaisseur de la paroi : 50 μm,
- longueur : 18 cm.

La surface interne destinée à venir en contact avec le sang ou le plasma est d'environ 230 cm$^2$.

Une solution de polyéthylèneimine (PEI P, BASF, de masse moléculaire moyenne 750.000 Daltons) est préparée dans un mélange eau/chlorure de sodium (0,15 M en NaCl).

La concentration en PEI est de 40 mg/l.

On fait circuler 8 ml de cette solution dans le compartiment interne du minidialyseur au débit de 4 ml/min. On fait ensuite circuler dans ce même compartiment pour rinçage 20 ml de solution aqueuse de NaCl à 0,15 M au débit de 4 ml/min.

Dans ces conditions, la quantité de PEI fixée à la membrane est d'environ 4 mg/m$^2$ (déterminée par dosage de la PEI en sortie du minidialyseur).

Les fibres ainsi traitées sont reglycérinées par circulation d'un mélange glycérol/eau (60/40 en masse), à raison de 20 ml avec un débit de 4 ml/min, et purgées à l'air afin d'éliminer l'excédent de mélange.

Ce minidialyseur est stérilisé par l'oxyde d'éthylène.

Remarque concernant l'évolution de la capacité ionique :

La capacité ionique globale initiale de la membrane AN69 est d'environ -200 μeq/g de polymère gonflé.

Si l'on considère la formule générale de la PEI -(CH$_2$-CH$_2$-NH)$_n$-, celle-ci contient environ 23 μmol de groupements amines par mg de PEI.

Dans l'hypothèse où tous les groupements amines sont ioniquement liés aux groupements sulfonates de l'AN69 (ce qui n'est pas possible physiquement), un calcul simple montre que la diminution de la capacité ionique globale de la membrane est négligeable (environ 0,8 %).

Aptitude de ce minidialyseur à prévenir l'activation de la phase contact :

Préalablement à ce test, le minidialyseur est rincé par circulation de 20 ml d'une solution de NaCl à 0,15 M au débit de 2 ml/min.

Un liquide biologique a été préparé, propre à stimuler la production de kallicréines au contact d'une membrane chargée négativement en surface. Le liquide biologique utilisé pour l'essai était constitué de plasma humain pauvre en plaquettes, dilué à 5 % dans du sérum physiologique additionné de citrate à titre d'anticoagulant (on note que les conditions du test utilisé sont éloignées des conditions d'utilisation d'un appareil pour circulation extracorporelle de sang : le taux de dilution est très élevé, le liquide choisi est du plasma et non du sang, le plasma est additionné de citrate, donc acidifié, alors qu'en dialyse, l'anticoagulant utilisé est de l'héparine. Ces conditions de test sont choisies à dessein car elles stimulent et amplifient l'activation de la phase contact). Ce liquide est mis en circulation en circuit ouvert dans le compartiment interne du minidialyseur à un débit de 2 ml/mn pendant 3 minutes. Les kallicréines plasmatiques ont été dosées dans des échantillons de liquide prélevés à intervalle de temps au moyen d'un test chromogénique classique, à partir du substrat S2302 de la société BIOGENIC.

Comme le montre le tableau 1, ce traitement modifie sensiblement les caractéristiques électriques de la membrane et a pour effet d'inhiber l'activation de la phase contact.

# EP 0 925 826 B1

Tableau 1

| Caractéristiques électriques et niveau d'activation de la phase contact des membranes AN69 et AN69 modifiée avec la PEI | | | | |
|---|---|---|---|---|
| Membrane | Potentiel zêta mV | Résistivité ohm. cm | Indice électro-cinétique I | Concentration de kallicréines formées en U/l |
| AN69 | -70 | 312 | 1,98 | 70 |
| AN69 modifiée avec la PEI | -1 | 312 | 0,5 | <10 |

Exemple 2

Un dialyseur (dénomination commerciale CRYSTAL 4000, fabriqué par HOSPAL), constitué de 57 compartiments sang parallèles et séparés par une membrane plane AN69 possède une surface susceptible d'être en contact avec le sang de 1,53 m$^2$. On fait subir à ce dialyseur les étapes suivantes:

-   circulation dans le compartiment sang de 2 litres de sérum physiologique à un débit de 200 ml/min (débit d'ultra-filtration de 22 ml/min).

-   circulation dans le compartiment sang de 500 ml d'une solution de PEI de masse moléculaire moyenne supérieure à 750000 Daltons à la concentration de 40 mg/l dans l'eau distillée à un débit de 200 ml/min (débit d'ultrafiltration de 22 ml/min).

-   rinçage par circulation dans le compartiment sang de 2 litres d'une solution de sérum physiologique à un débit de 200 ml/min (débit d'ultrafiltration de 22 ml/min).

-   reglycérinage par circulation dans le compartiment sang de 1 litre d'une solution glycérol/eau (60/40 en masse) à un débit de 200 ml/min (débit d'ultra-filtration de 22 ml/min).

Le potentiel Zêta « Z » est calculé à partir de la mesure du potentiel « E » selon les conditions énoncées dans la description.

Ensuite, après purge à l'air, le dialyseur est stérilisé par irradiation gamma (25 à 36 kGy).

Après stockage, et après rinçage pendant 10 minutes par circulation d'une solution de NaCl (0,15 M) à 200 ml/min, le dialyseur est testé au niveau de son aptitude à générer les kallicréines au contact de plasma dilué suivant la méthode décrite dans l'exemple 1 (le débit du liquide biologique dans le compartiment interne du dialyseur est dans ce cas de 100 ml/mn).

Tableau 2

| Potentiel Zêta « Z» et niveau d'activation de la phase contact des dialyseurs AN69 et AN69 modifié par la PEI | | |
|---|---|---|
| Dialyseur | Potentiel Zêta mV | Concentration de kallicréines formées U/l |
| CRYSTAL 4000 | - 72 | 75 |
| CRYSTAL 4000 traité PEI | + 2,9 | <10 |

Exemple 3

Une membrane plane en AN69 (épaisseur 20 µm) est traitée par pulvérisation de PEI de masse moléculaire moyenne supérieure à 750000 Daltons à la concentration de 5 g/kg dans un mélange glycérol/eau 60/40 en masse. La quantité déposée est de l'ordre de 9 mg/m$^2$ de membrane.

Avec cette membrane, on assemble un dialyseur comportant 39 compartiments sang parallèles et séparés par la membrane plane en AN69, de manière à ce que la face traitée soit celle en contact du sang. La surface de la membrane destinée à venir en contact du sang est d'environ 1,04 m$^2$.

Le potentiel Zêta « Z » est calculé à partir de la mesure du potentiel « E » selon les conditions énoncées dans la description. Le potentiel d'écoulement d'un tel dialyseur est égal à +10 µV/mm Hg alors que celui d'un dialyseur du même type sans PEI est égal à - 47 µV/mm Hg.

Ensuite, après stérilisation par irradiation gamma (36 K Gy), le dialyseur est testé au niveau de son aptitude à générer les kallicréines au contact de plasma dilué suivant la méthode décrite dans l'exemple 1 (le débit du liquide biologique dans le compartiment interne du dialyseur est dans ce cas de 100 ml/mn).

Tableau 3

| Potentiel Zêta « Z » et niveau d'activation de la phase contact des dialyseurs AN69 et AN69 modifié par la PEI | | |
| --- | --- | --- |
| **Dialyseur** | **Potentiel Zêta mV** | **Concentration de kallicréines formées U/l** |
| AN69 | - 70 | 59 |
| AN69 modifié par pulvérisation de PEI | 14,8 | < 10 |

Exemple 4

Un dialyseur comprenant environ 8500 fibres creuses AN69 a été assemblé. La surface de la membrane destinée à venir au contact du sang ou du plasma est d'environ 1,34 m$^2$.

On prépare 200 ml d'une solution contenant 5 g/kg de PEI (masse moléculaire moyenne supérieure à 750000 Daltons) dans un mélange glycérol/eau 60/40 massique. On fait circuler cette solution dans le compartiment interne du dialyseur en circuit ouvert au débit de 200 ml/min. Le compartiment sang, ou compartiment interne, est délimité par l'intérieur des fibres et deux embouts munis chacun d'une tubulure d'accès, fixés aux extrémités du boîtier du dialyseur. Le potentiel Zêta « Z » est calculé à partir de la mesure du potentiel « E » selon les conditions énoncées dans la description. Le potentiel d'écoulement mesuré avec ce dialyseur est égal à + 2,7 µV/mm Hg (-22 µV/mm Hg dans le cas d'un dialyseur du même type sans PEI).

Ensuite, après stérilisation par irradiation gamma (36 K Gy), le dialyseur est testé au niveau de son aptitude à générer les kallicréines au contact de plasma dilué suivant la méthode décrite dans l'exemple 1.

Après rinçage pendant 10 minutes par circulation d'une solution de NaCI (0,15 M) à 200 ml/min, on fait subir au dialyseur le test biologique décrit dans l'exemple 1 (le débit du liquide biologique dans le compartiment interne du dialyseur est dans ce cas de 100 ml/mn).

Le tableau 4 ci-après donne les résultats des différentes mesures effectuées et le niveau d'activation de la phase contact atteint.

| **Dialyseur** | **Potentiel Zêta mV** | **Concentration de kallicréines formées U/l** |
| --- | --- | --- |
| AN69 | - 72 | 70 |
| N69 modifié par de la PEI | 9,3 | < 10 |

La capacité ionique globale des fibres creuses en AN69, sans PEI, est de -174 µeq/g de polymère gonflé et celle des fibres creuses en AN69 modifié par de la PEI est de - 163 µeq/g de polymère gonflé.

**Revendications**

**1.** Utilisation d'un polymère neutre ou cationique pour prévenir l'activation de la phase contact du sang ou du plasma venant au contact d'une membrane semi-perméable à base de polyacrylonitrile porteur de charges négatives fixes, prévue dans un appareil pour le traitement du sang ou du plasma par circulation extracorporelle, selon laquelle :

- le polymère, neutre ou cationique, est combiné à cette membrane semi-perméable, avant ou après formation de la membrane, et avant stérilisation de la membrane ;

- la capacité ionique globale de la membrane semi-perméable à base de polyacrylonitrile porteur de charges négatives fixes et renfermant un polymère neutre ou cationique, est inférieure à - 30 µeq/g de polymère gouflé ;

- la capacité ionique globale de la membrane semi-perméable à base de polyacrylonitrile porteur de charges négatives fixes et renfermant un polymère neutre ou cationique est, en valeur absolue, au plus de 10% inférieure à la capacité ionique globale de la même membrane semi-perméable, exempte de polymère neutre ou cationique ;

- la membrane semi-perméable à base de polyacrylonitrile porteur de charges négatives fixes et renfermant un polymère neutre ou cationique, vérifie, avant stérilisation, l'une ou l'autre des deux caractéristiques électriques qui suivent :

* l'indice électrocinétique «I» est inférieur ou égal à 0,8, «I» correspondant au logarithme, en base 10, du rapport |Z|/R, où «Z» est le potentiel Zêta exprimé en microvolt, en valeur absolue, de la surface de la membrane destinée à venir au contact du sang ou du plasma, et où «R» est la résistivité électrique de la membrane exprimée en ohm.centimètre,

* ou, le potentiel Zêta « Z » est positif et compris entre 0 et + 15 mV, bornes non incluses.

2. Utilisation selon la revendication 1, **caractérisée en ce que** l'indice électrocinétique «I» de la membrane semi-perméable à base de polyacrylonitrile porteur de charges négatives fixes et renfermant un polymère neutre ou cationique est au plus égal à 0,7 ou inférieur.

3. Utilisation selon la revendication 2, **caractérisée en ce que** l'indice électrocinétique «I» de la membrane semi-perméable à base de polyacrylonitrile porteur de charges négatives fixes et renfermant un polymère neutre ou cationique est au plus égal à 0,6 ou inférieur.

4. Utilisation selon la revendication 1, **caractérisée en ce que** le potentiel Zêta « Z » est positif, est supérieur ou égal à +1 mV et inférieur strictement à + 15 mV.

5. Utilisation selon la revendication 1, **caractérisée en ce que** le potentiel Zêta « Z» est positif, est supérieur ou égal à +6 mV et inférieur strictement à +15 mV.

6. Utilisation selon la revendication 1 à 5, **caractérisée en ce que** la capacité ionique globale de la membrane semi-perméable à base de polyacrylonitrile porteur de charges négatives fixes et renfermant un polymère neutre ou cationique, est inférieure à -50 μeq/g de polymère gonflé.

7. Utilisation selon l'une des revendications précédentes, **caractérisée en ce que** la capacité ionique globale de la membrane semi-perméable à base de polyacrylonitrile porteur de charges négatives fixes et renfermant un polymère neutre ou cationique, est, en valeur absolue, au plus de 1% inférieure à la capacité ionique globale de la même membrane semi-perméable, exempte de polymère neutre ou cationique.

8. Utilisation selon l'une des revendications précédentes, **caractérisée en ce que** le polymère est neutre et a une masse moléculaire moyenne supérieure à 40.000 Daltons.

9. Utilisation selon la revendication 8, **caractérisée en ce que** le polymère neutre a une masse moléculaire moyenne supérieure à 100.000 Daltons.

10. Utilisation selon la revendication 8 ou 9, **caractérisée en ce que** le polymère neutre est choisi dans le groupe constitué par des polyvinylpyrrolidones et des polyéthylèneglycols.

11. Utilisation selon l'une des revendications 1 à 7, **caractérisée en ce que** le polymère est cationique et a une masse moléculaire moyenne supérieure à 25.000 Daltons.

12. Utilisation selon la revendication 11, **caractérisée en ce que** le polymère cationique a une masse moléculaire moyenne supérieure à 100.000 Daltons.

13. Utilisation selon la revendication 11, **caractérisée en ce que** le polymère cationique est choisi parmi les polyamines, de préférence parmi les polyéthylèneimines.

14. Utilisation selon la revendication 11, **caractérisée en ce que** le polymère cationique est choisi parmi les diméthylaminoéthyles dextran.

15. Utilisation selon l'une des revendications précédentes, **caractérisée en ce que** le polyacrylonitrile constitutif de la membrane semi-perméable est un copolymère de l'acrylonitrile et d'au moins un monomère anionique ou anionisable, renfermant, le cas échéant, des unités provenant d'au moins un autre monomère à insaturation oléfinique capable d'être copolymérisé avec l'acrylonitrile.

**16.** Utilisation selon la revendication 15, **caractérisée en ce que** le polyacrylonitrile est un copolymère de l'acrylonitrile et d'un comonomère anionique ou anionisable, oléfiniquement insaturé, et porteurs de groupements anioniques choisis parmi les groupes sulfonate, carboxyle, phosphate, phosphonate et sulfate.

**17.** Utilisation selon la revendication 16, **caractérisée en ce que** le comonomère est le méthallylsulfonate de sodium.

**18.** Utilisation selon l'une des revendications 1 à 14, **caractérisée en ce que** le polyacrylonitrile constitutif de la membrane semi-perméable est un copolymère de l'acrylonitrile et d'au moins un monomère non ionique et non ionisable, renfermant, le cas échéant, des unités provenant d'au moins un autre monomère à insaturation oléfinique capable d'être copolymérisé avec l'acrylonitrile.

**19.** Procédé de fabrication d'un appareil pour le traitement du sang ou du plasma par circulation extra-corporelle permettant de prévenir l'activation de la phase contact et comprenant une membrane semi-perméable, sous la forme d'une membrane plane ou d'un faisceau de fibres creuses, à base de polyacrylonitrile porteur de charges négatives fixes, **caractérisé en ce qu'**il comprend les étapes suivantes:

.    préparer une solution constituée de :

-    au moins un polyacrylonitrile porteur de charges négatives fixes ;

-    au moins un polymère neutre,:

-    au moins un solvant dudit polyacrylonitrile et du polymère neutre,

-    éventuellement, au moins un non solvant du polyacrylonitrile,

.    extruder cette solution pour former une fibre creuse ou une membrane plane ;
.    simultanément dans le cas de la préparation d'une fibre creuse ou après l'extrusion dans le cas de la formation d'une membrane plane, solidifier la membrane obtenue par un procédé d'inversion de phase par contact partiel ou total du produit extrudé avec un fluide, liquide ou gazeux, et chimiquement inerte vis-à-vis desdits polymères ;
.    laver la membrane plane ou la fibre creuse obtenue ;
.    éventuellement, glycériner la membrane plane ou la fibre creuse ;
.    préparer une membrane semi-perméable à partir de la membrane plane ou conformer un faisceau de fibres creuses à partir de la fibre creuse ;
.    monter la membrane plane ou le faisceau de fibres creuses dans un boîtier et, le cas échéant, fixer des embouts au boîtier ;
.    stériliser l'appareil médical obtenu,

et **en ce que** la quantité de polymère neutre est ajustée afin que :

.    la capacité ionique globale de la membrane semi-perméable, à base de polyacrylonitrile porteur de charges négatives fixes et renfermant un polymère neutre, soit inférieure à - 30 $\mu$eq/g de polymère gouflé ;

.    la capacité ionique globale de la membrane semi-perméable, à base de polyacrylonitrile porteur de charges négatives fixes et renfermant un polymère neutre, soit, en valeur absolue, au plus de 10% inférieure à la capacité ionique globale de la même membrane semi-perméable, exempte de polymère neutre ;

.    la membrane semi-perméable, à base de polyacrylonitrile porteur de charges négatives fixes et renfermant un polymère neutre vérifie, avant stérilisation, l'une ou l'autre des caractéristiques électriques qui suivent :

*    l'indice électrocinétique «I» de la membrane semi-perméable renfermant un polymère neutre, qui est égal à $Log_{10}(|Z|/R)$ c'est-à-dire au logarithme, en base 10, du rapport $|Z|/R$ du potentiel Zêta «Z», en valeur absolue, exprimé en microvolt, de la surface de la membrane destinée à venir au contact du sang ou du plasma, sur la résistivité électrique «R» de la membrane exprimée en ohm.centimètre, est inférieur ou égal à 0,8,

\* ou, le potentiel Zêta « Z» est positif et varie entre 0 et + 15 mV, bornes non incluses.

**20.** Procédé de fabrication d'un appareil pour le traitement du sang ou du plasma par circulation extra-corporelle permettant de prévenir l'activation de la phase contact et comprenant une membrane semi-perméable, sous la forme d'une membrane plane ou d'un faisceau de fibres creuses, à base de polyacrylonitrile porteur de charges négatives fixes, **caractérisé en ce qu'**il comprend les étapes suivantes:

(a) préparer une membrane plane ou une fibre creuse, éventuellement glycérinée, à partir d'une solution de polyacrylonitrile porteur de charges négatives;
(b) assembler les divers composants de l'appareil, en particulier monter la membrane semi-perméable ou un faisceau de fibres creuses dans un boîtier et réaliser les embouts de ce boîtier ;
(c) simultanément ou successivement, éventuellement déglycériner la membrane semi-perméable et préparer une solution contenant le polymère cationique à l'état dissous et porter cette solution au contact de la surface de la membrane semi-perméable destinée à être mise en contact avec le sang, l'étape (c) pouvant être réalisée avant ou après l'étape (b),
(d) dans le cas où l'étape (c) précitée a été réalisée postérieurement à l'étape (b), purger l'appareil de la solution contenant le polymère cationique ;
(e) éventuellement, rincer la membrane semi-perméable pour éliminer l'excédent de polymère cationique non fixé et, éventuellement, reglycériner la membrane semi-perméable ;
(f) stériliser l'appareil médical,

et **en ce que** la quantité de polymère cationique est ajustée afin que :

. la capacité ionique globale de la membrane semi-perméable, à base de polyacrylonitrile porteur de charges négatives fixes et renfermant un polymère cationique, soit inférieure à 30 µeq/g de polymère gouflé ;

. la capacité ionique globale de la membrane semi-perméable, à base de polyacrylonitrile porteur de charges négatives fixes et renfermant un polymère cationique, soit, en valeur absolue, au plus de 10% inférieure à la capacité ionique globale de la même membrane semi-perméable, exempte de polymère cationique;

. la membrane semi-perméable, à base de polyacrylonitrile porteur de charges négatives fixes et renfermant un polymère cationique, vérifie, avant stérilisation, l'une ou l'autre des caractéristiques électriques qui suivent :

\* l'indice électrocinétique «I» de la membrane semi-perméable renfermant un polymère cationique, qui est égal à $Log_{10}(|Z|/R)$ c'est-à-dire au logarithme, en base 10, du rapport $|Z|/R$ du potentiel Zêta «Z», en valeur absolue, exprimé en microvolt, de la surface de la membrane destinée à venir au contact du sang ou du plasma, sur la résistivité électrique «R» de la membrane exprimée en ohm.centimètre, est inférieur ou égal à 0,8,

\* ou, le potentiel Zêta « Z » est positif et varie entre 0 et +15 mV, bornes non incluses.

**21.** Procédé de fabrication d'un appareil pour le traitement du sang ou du plasma par circulation extra-corporelle permettant de prévenir l'activation de la phase contact et comprenant une membrane semi-perméable, sous la forme d'une membrane plane, à base de polyacrylonitrile porteur de charges négatives fixes, **caractérisé en ce qu'**il comprend les étapes suivantes :

(a) préparer une membrane plane, éventuellement glycérinée, à partir d'une solution de polyacrylonitrile porteur de charges négatives;
(b) simultanément ou successivement, éventuellement déglycériner la membrane semi-perméable et préparer une solution contenant le polymère cationique ou neutre à l'état dissous et pulvériser cette solution sur la surface de la membrane semi-perméable destinée à être mise en contact avec le sang ;
(c) assembler les divers composants de l'appareil, en particulier monter la membrane semi-perméable dans un boîtier et réaliser les embouts de ce boîtier ;
(d) éventuellement, rincer la membrane semi-perméable pour éliminer l'excédent de polymère cationique non fixé et, éventuellement, reglycériner la membrane semi-perméable ;
(f) stériliser l'appareil médical,

et **en ce que** la quantité de polymère cationique ou neutre est ajustée afin que :.

. la capacité ionique globale de la membrane semi-perméable, à base de polyacrylonitrile porteur de charges négatives fixes et renfermant un polymère cationique ou neutre, est inférieure à - 30 µeq/g de polymère gouflé ;

. la capacité ionique globale de la membrane semi-perméable, à base de polyacrylonitrile porteur de charges négatives fixes et renfermant un polymère cationique ou neutre, est, en valeur absolue, au plus de 10% inférieure à la capacité ionique globale de la même membrane semi-perméable, exempte de polymère cationique ou neutre;

. la membrane semi-perméable, à base de polyacrylonitrile porteur de charges négatives fixes et renfermant un polymère neutre ou cationique, vérifie, avant stérilisation, l'une ou l'autre des caractéristiques électriques qui suivent :

\* l'indice électrocinétique «I» de la membrane semi-perméable renfermant un polymère cationique ou neutre, qui est égal à $Log_{10}(|Z|/R)$ c'est-à-dire au logarithme, en base 10, du rapport $|Z|/R$ du potentiel Zêta «Z», en valeur absolue, exprimé en microvolt, de la surface de la membrane destinée à venir au contact du sang ou du plasma, sur la résistivité électrique «R» de la membrane exprimée en ohm.centimètre, est inférieur ou égal à 0,8,

\* ou, le potentiel Zêta « Z » est positif et varie entre 0 et + 15 mV bornes non incluses.

22. Appareil pour le traitement du sang où du plasma par circulation extracorporelle, stérilisé et prêt à l'emploi, permettant de prévenir l'activation de la phase contact et comprenant une membrane semi-perméable, sous la forme d'une membrane plane ou d'un faisceau de fibres creuses, à base de polyacrylonitrile porteur de charges négatives fixes, **caractérisé en ce que** avant ou après formation de la membrane semi-perméable, et avant stérilisation, au moins un polymère neutre ou cationique est incorporé à la membrane semi-perméable, en une quantité appropriée de sorte que :

- la capacité ionique globale de la membrane semi-perméable à base de polyacrylonitrile porteur de charges négatives fixes et renfermant un polymère neutre ou cationique, est inférieure à - 30 µeq/g de polymère gouflé ;

- la capacité ionique globale de la membrane semi-perméable à base de polyacrylonitrile porteur de charges négatives fixes et renfermant un polymère neutre ou cationique, est, en valeur absolue, au plus de 10% inférieure à la capacité ionique globale de la même membrane semi-perméable, exempte de polymère neutre ou cationique ;

- la membrane semi-perméable à base de polyacrylonitrile porteur de charges négatives fixes et renfermant un polymère neutre ou cationique, vérifie, avant stérilisation, l'une ou l'autre des caractéristiques électriques qui suivent :

\* l'indice électrocinétique «I» de la membrane, renfermant un polymère neutre ou cationique est inférieur ou égal à 0,8, «I» étant égal au logarithme, en base 10, du rapport $|Z|/R$, où «Z» est le potentiel Zêta, en valeur absolue, exprimé en microvolt, de la surface de la membrane destinée à venir au contact du sang ou du plasma, et où «R» est la résistivité électrique de la membrane exprimée en ohm.centimètre,

\* ou, le potentiel Zêta « Z » est positif et est compris entre 0 et +15 mV, bornes non incluses.

**Patentansprüche**

1. Verwendung eines neutralen oder kationischen Polymers zur Verhinderung der Aktivierung der Blut- oder Plasmakontaktphase in Berührung mit einer teildurchlässigen Membran auf Polyacrylonitrilbasis, die Träger fester negativer Ladungen und für ein Gerät zur Behandlung von Blut oder Plasma durch extrakorporelle Zirkulation vorgesehen ist, gemäß der:

- das neutrale oder kationische Polymer mit dieser teildurchlässigen Membran verbunden wird, vor oder nach der Bildung der Membran, und vor der Sterilisation der Membran;

- die globale ionische Kapazität der teildurchlässigen Membran auf Polyacrylonitrilbasis, die ein Träger fester, negativer Ladungen ist und ein neutrales oder kationisches Polymer enthält, geringer als -30 µeq/g des aufgeschäumten Polymers ist;

- der Betrag der globalen ionischen Kapazität der teildurchlässigen Membran auf Polyacrylonitrilbasis, die ein Träger fester, negativer Ladungen ist und ein neutrales oder kationisches Polymer enthält, höchstens 10% geringer ist als die globale ionische Kapazität derselben teildurchlässigen Membran, befreit vom neutralen oder kationischen Polymer;

- die teildurchlässige Membran auf Polyacrylonitrilbasis, die ein Träger fester, negativer Ladungen ist und ein neutrales oder kationisches Polymer enthält, vor der Sterilisation die eine oder andere der folgenden elektrischen Eigenschaften aufweist:

  - der elektrokinetische Index "I" ist niedriger oder gleich 0,8, wobei "I" dem Logarithmus der Proportion |Z|/R zur Basis 10 entspricht, und "Z" der Betrag des Zeta-Potentials, ausgedrückt in Mikrovolt, der Oberfläche der Membran ist, die in Berührung mit dem Blut oder Plasma kommen soll, und "R" die elektrische Resistivität der Membran in Ohm-Zentimeter darstellt

  - wobei das Zeta-Potenzial "Z" positiv ist und zwischen 0 und + 15 mV, Grenzwerte nicht eingeschlossen, liegt

2. Verwendung gemäß Anspruch 1, **dadurch gekennzeichnet dass** der elektrokinetische Index "I" der teildurchlässigen Membran auf Polyacrylonitrilbasis, die ein Träger fester, negativer Ladungen ist und ein neutrales oder kationisches Polymer enthält, höchstens 0,7 entspricht oder weniger.

3. Verwendung gemäß Anspruch 2, **dadurch gekennzeichnet dass** der elektrokinetische Index "I" der teildurchlässigen Membran auf Polyacrylonitrilbasis, die ein Träger fester, negativer Ladungen ist und ein neutrales oder kationisches Polymer enthält, höchstens 0,6 entspricht oder weniger.

4. Verwendung gemäß Anspruch 1, **dadurch gekennzeichnet dass** das Zeta-Potential "Z" positiv ist sowie größer oder gleich + 1 mV und strikt geringer als + 15 mV.

5. Verwendung gemäß Anspruch 1, **dadurch gekennzeichnet dass** das Zeta-Potential "Z" positiv ist sowie größer oder gleich + 6 mV und strikt geringer als + 15 mV.

6. Verwendung gemäß Anspruch 1 bis 5, **dadurch gekennzeichnet dass** die globale ionische Kapazität der teildurchlässigen Membran auf Polyacrylonitrilbasis, die ein Träger fester, negativer Ladungen ist und ein neutrales oder kationisches Polymer enthält, geringer als -50 µeg/g des aufgeschäumten Polymers ist.

7. Verwendung gemäß einem der o.g. Ansprüche, **dadurch gekennzeichnet dass** der Betrag der globalen ionischen Kapazität der teildurchlässigen Membran auf Polyacrylonitrilbasis, die ein Träger fester, negativer Ladungen ist und ein neutrales oder kationisches Polymer enthält, höchstens 1% geringer ist als die globale ionische Kapazität derselben teildurchlässigen Membran, befreit vom neutralen oder kationischen Polymer.

8. Verwendung gemäß einem der o.g. Ansprüche, **dadurch gekennzeichnet dass** das Polymer neutral ist und eine durchschnittliche molekulare Masse von über 40.000 Daltons besitzt.

9. Verwendung gemäß Anspruch 8, **dadurch gekennzeichnet dass** das neutrale Polymer eine durchschnittliche molekulare Masse von über 100.000 Daltons besitzt.

10. Verwendung gemäß Anspruch 8 oder 9, **dadurch gekennzeichnet dass** das neutrale Polymer aus der Klasse der Polyvinylpyrrolidone und der Polethylenglykole gewählt wird.

11. Verwendung gemäß einem der Ansprüche 1 bis 7, **dadurch gekennzeichnet dass** das Polymer kationisch ist und eine durchschnittliche molekulare Masse von über 25.000 Daltons besitzt.

12. Verwendung gemäß Anspruch 11, **dadurch gekennzeichnet dass** das kationische Polymer eine durchschnittliche molekulare Masse von über 100.000 Daltons besitzt.

**13.** Verwendung gemäß Anspruch 11, **dadurch gekennzeichnet dass** das kationische Polymer aus der Klasse der Polyamine und vorzugsweise unter den Polyethyleniminen gewählt wird.

**14.** Verwendung gemäß Anspruch 11, **dadurch gekennzeichnet dass** das kationische Polymer ein Dimethlaminoethyl Dextran ist.

**15.** Verwendung gemäß einem der o.g. Ansprüche, **dadurch gekennzeichnet dass** das Polyacryonitril, das die teildurchlässige Membran bildet, ein Copolymer aus Acrylonitril und mindestens ein anionisches oder anionisierbares Monomer ist, das ggf. Einheiten enthält, die von mindestens einem anderen olefinisch ungesättigten Monomer stammen, das mit Acrylonitril copolymerisiert werden kann.

**16.** Verwendung gemäß Anspruch 15, **dadurch gekennzeichnet dass** das Polyacrylonitril ein Copolymer aus Acrylonitril und aus einem olefinisch ungesättigten, anionischen oder anionisierbaren Comonomer ist und Träger anionischer Gruppierungen ist, die aus der Klasse der Sulfonate, Carboxyle, Phosphate, Phosphonate und Sulfate gewählt werden.

**17.** Verwendung gemäß Anspruch 16, **dadurch gekennzeichnet dass** das Comonomer ein Natriummethallylsulfonat ist.

**18.** Verwendung gemäß einem der Ansprüche 1 bis 14, **dadurch gekennzeichnet dass** das Polyacrylonitril, das die teildurchlässige Membran bildet, ein Copolymer aus Acrylonitril und mindestens ein nicht ionisches und nicht ionisierbares Monomer ist, das ggf. Einheiten enthält, die von mindestens einem anderen olefinisch ungesättigten Monomer stammen, das mit Acrylonitril copolymerisiert werden kann.

**19.** Verfahren zur Herstellung eines Geräts für die Behandlung von Blut oder Plasma durch extrakorporelle Zirkulation, mit dem es möglich ist, die Aktivierung der Kontaktphase zu verhindern, und das eine teildurchlässige Membran auf Polyacrylonitrilbasis in Form einer flachen Membran oder eines Bündels Hohlfasern enthält, die Träger fester, negativer Ladungen ist, und durch die folgenden Phasen **gekennzeichnet** wird:

- eine Lösung zubereiten, die folgende Bestandteile enthält:

    - mindestens ein Polyacryonitril, Träger fester, negativer Ladungen
    - mindestens ein neutrales Polymer
    - mindestens ein Lösungsmittel des besagten Polyacrylonitrils und des neutralen Polymers
    - eventuell mindestens ein das Polyacrylonitril nicht lösendes Mittel

- diese Lösung extrudieren, um eine Hohlfaser oder eine flache Membran zu bilden;
- die so erhaltene Membran gleichzeitig - im Falle der Zubereitung einer Hohlfaser - oder danach - im Falle der Bildung einer flachen Membran - verfestigen, und zwar mit einem Inversionsverfahren der Phase durch den partiellen oder vollständigen Kontakt des extrudierten Produkts mit einer flüssigen oder gasförmigen Flüssigkeit, die gegenüber den besagten Polymeren chemisch inert ist;
- die erhaltene flache Membran oder Hohlfaser waschen
- die flache Membran oder die Hohlfaser eventuell glycerinieren
- eine teildurchlässige Membran aus der flachen Membran bilden oder ein Bündel Hohlfasern aus der Hohlfaser formen,
- die flache Membran oder das Bündel Hohlfasern in ein Gehäuse montieren und ggf. Endverschlüsse am Gehäuse anbringen;
- das so erhaltene medizinische Gerät sterilisieren,

und wobei die Menge des neutralen Polymers angepasst wird, damit:

- die globale ionische Kapazität der teildurchlässigen Membran auf Polyacrylonitrilbasis, die ein Träger fester, negativer Ladungen ist und ein neutrales Polymer enthält, unter -30 µeq/g des aufgeschäumten Polymers liegt;

- der Betrag der globalen ionischen Kapazität der teildurchlässigen Membran auf Polyacrylonitrilbasis, die ein Träger fester, negativer Ladungen ist und ein neutrales Polymer enthält, höchstens 10% geringer als die globale ionische Kapazität derselben teildurchlässigen Membran, befreit vom neutralen Polymer, ist;

- die teildurchlässige Membran auf Polyacrylonitrilbasis, die ein Träger fester, negativer Ladungen ist und ein neutrales Polymer enthält, vor der Sterilisation die eine oder andere der folgenden elektrischen Eigenschaften aufweist:

  - der elektrokinetische Index "I" der teildurchlässigen Membran, die ein neutrales Polymer enthält und $\text{Log}_{10}$ ($|Z|/R$) entspricht, d.h. dem Logarithmus der Proportion $|Z|/R$ zur Basis 10 des Betrags des in Mikrovolt ausgedrückten Zeta-Potentials "Z" der Oberfläche der Membran, die in Berührung mit dem Blut oder Plasma kommen soll, auf der elektrischen Resistivität "R" der Membran, ausgedrückt in Ohm-Zentimeter, ist niedriger oder gleich 0,8,
  - wobei das Zeta-Potenzial "Z" positiv ist und zwischen 0 und + 15 mV, Grenzwerte nicht eingeschlossen, variiert.

**20.** Verfahren zur Herstellung eines Geräts für die Behandlung von Blut oder Plasma durch extrakorporelle Zirkulation, mit dem es möglich ist, die Aktivierung der Kontaktphase zu verhindern, und das eine teildurchlässige Membran auf Polyacrylonitrilbasis in Form einer flachen Membran oder eines Bündels Hohlfasern enthält, die Träger fester, negativer Ladungen ist, und durch die folgenden Phasen **gekennzeichnet** wird:

  (a) Vorbereitung einer flachen Membran oder einer Hohlfaser, die eventuell glyceriniert wird, ausgehend von einer Lösung aus Polyacrylonitril, die Träger negativer Ladungen ist.
  (b) Assemblierung der verschiedenen Komponenten des Geräts, insbesondere Montage der teildurchlässigen Membran oder des Hohlfaserbündels in einem Gehäuse und Fertigung der Endverschlüsse dieses Gehäuses.
  (c) Gleichzeitig oder danach, die teildurchlässige Membran eventuell deglycerinieren und eine Lösung zubereiten, die ein kationisches Polymer in gelöstem Zustand enthält, und diese Lösung in Berührung mit der Oberfläche der teildurchlässigen Membran bringen, die in Kontakt mit dem Blut kommen soll; die Phase (c) kann vor oder nach der Phase (b) stattfinden.
  (d) Im Falle dass die o.g. Phase (c) nach der Phase (b) durchgeführt wurde, die Flüssigkeit, die das kationische Polymer enthält, aus dem Gerät entfernen.
  (e) Die teildurchlässige Membran eventuell abspülen, um das überschüssige, nicht fixierte kationische Polymer zu entfernen und die teildurchlässige Membran eventuell erneut glycerinieren.
  (f) Das medizinische Gerät sterilisieren.

und wobei die Menge des neutralen Polymers angepasst wird, damit:

- die globale ionische Kapazität der teildurchlässigen Membran auf Polyacrylonitrilbasis, die ein Träger fester, negativer Ladungen ist und ein kationisches Polymer enthält, unter -30 µeq/g des aufgeschäumten Polymers liegt;

- der Betrag der globalen ionischen Kapazität der teildurchlässigen Membran auf Polyacrylonitrilbasis, die ein Träger fester, negativer Ladungen ist und ein kationisches Polymer enthält, höchstens 10% geringer als die globale ionische Kapazität derselben teildurchlässigen Membran, befreit vom kationischen Polymer, ist;

- die teildurchlässige Membran auf Polyacrylonitrilbasis, die ein Träger fester, negativer Ladungen ist und ein kationisches Polymer enthält, vor der Sterilisation die eine oder andere der folgenden elektrischen Eigenschaften aufweist:

  - der elektrokinetische Index "I" der Membran, die ein kationisches Polymer enthält, $\text{Log}_{10}$ ($|Z|/R$) entspricht, d.h. dem Logarithmus der Proportion $|Z|/R$ zur Basis 10 des Betrags des in Mikrovolt ausgedrückten Zeta-Potentials "Z" der Oberfläche der Membran, die in Berührung mit dem Blut oder Plasma kommen soll, auf der elektrischen Resistivität "R" der Membran, ausgedrückt in Ohm-Zentimetern, ist niedriger oder gleich 0,8,

  - wobei das Zeta-Potential "Z" positiv ist und zwischen 0 und + 15 mV, Grenzwerte nicht eingeschlossen, variiert.

**21.** Verfahren zur Herstellung eines Geräts für die Behandlung von Blut oder Plasma durch extrakorporelle Zirkulation, mit dem es möglich ist, die Aktivierung der Kontaktphase zu verhindern, und das eine teildurchlässige Membran auf Polyacrylonitrilbasis in Form einer flachen Membran auf Polyacrylonitrilbasis, die Träger fester, negativer Ladungen ist, und durch die folgenden Phasen **gekennzeichnet** wird:

(a) Vorbereitung einer flachen Membran oder einer Hohlfaser, die eventuell glyceriniert wird, ausgehend von einer Lösung aus Polyacrylonitril, die Träger negativer Ladungen ist.

(b) Gleichzeitig oder danach, die teildurchlässige Membran eventuell deglycerinieren und eine Lösung zubereiten, die ein kationisches oder neutrales Polymer in gelöstem Zustand enthält, und diese Lösung auf der Oberfläche der teildurchlässigen Membran pulverisieren, die in Kontakt mit dem Blut kommen soll.

(c) Assemblierung der verschiedenen Komponenten des Geräts, insbesondere Montage der teildurchlässigen Membran oder des Hohlfaserbündels in einem Gehäuse und Fertigung der Endverschlüsse dieses Gehäuses.

(d) Die teildurchlässige Membran eventuell abspülen, um das überschüssige, nicht fixierte kationische Polymer zu entfernen und die teildurchlässige Membran eventuell erneut glycerinieren.

(e) Das medizinische Gerät sterilisieren.

und wobei die Menge des kationischen oder neutralen Polymers angepasst wird, damit:

- die globale ionische Kapazität der teildurchlässigen Membran auf Polyacrylonitrilbasis, die ein Träger fester, negativer Ladungen ist und ein neutrales oder kationisches Polymer enthält, unter -30 $\mu$eq/g des aufgeschäumten Polymers liegt;

- der Betrag der globalen ionischen Kapazität der teildurchlässigen Membran auf Polyacrylonitrilbasis, die ein Träger fester, negativer Ladungen ist und ein neutrales oder kationisches Polymer enthält, höchstens 10% geringer als die globale ionische Kapazität derselben teildurchlässigen Membran, befreit vom neutralen oder kationischen Polymer, ist;

- die teildurchlässige Membran auf Polyacrylonitrilbasis, die ein Träger fester, negativer Ladungen ist und ein neutrales oder kationisches Polymer enthält, vor der Sterilisation die eine oder andere der folgenden elektrischen Eigenschaften aufweist:

  - der elektrokinetische Index "I" der Membran, die ein neutrales oder kationisches Polymer enthält, $\text{Log}_{10}$ ($|Z|$ /R ) entspricht, d.h. dem Logarithmus der Proportion $|Z|$/R zur Basis 10 des Betrags des in Mikrovolt ausgedrückten Zeta-Potentials "Z" der Oberfläche der Membran, die in Berührung mit dem Blut oder Plasma kommen soll, auf der elektrischen Resistivität "R" der Membran, ausgedrückt in Ohm-Zentimetern, ist niedriger oder gleich 0,8,

  - wobei das Zet-Potential "Z" positiv ist und zwischen 0 und + 15 mV, Grenzwerte nicht eingeschlossen, variiert.

22. Steriles und anwendungsbereites Gerät zur Behandlung von Blut oder Plasma durch extrakorporelle Zirkulation, mit dem die Aktivierung der Kontaktphase verhindert werden kann und das eine teildurchlässige Membran auf Polyacrylonitrilbasis in Form einer flachen Membran oder eines Hohlfaserbündels enthält, die Träger fester, negativer Ladungen ist und **dadurch gekennzeichnet** wird, dass vor oder nach der Bildung der teildurchlässigen Membran und vor der Sterilisation mindestens ein neutrales oder kationisches Polymer in die teildurchlässige Membran eingefügt wurde, und zwar in angemessener Menge, so dass:

- die globale ionische Kapazität der teildurchlässigen Membran auf Polyacrylonitrilbasis, die ein Träger fester, negativer Ladungen ist und ein neutrales oder kationisches Polymer enthält, unter -30 $\mu$eq/g des aufgeschäumten Polymers liegt;

- der Betrag der globalen ionischen Kapazität der teildurchlässigen Membran auf Polyacrylonitrilbasis, die ein Träger fester, negativer Ladungen ist und ein neutrales oder kationisches Polymer enthält, höchstens 10% geringer als die globale ionische Kapazität derselben teildurchlässigen Membran, befreit vom neutralen oder kationischen Polymer, ist ;

- die teildurchlässige Membran auf Polyacrylonitrilbasis, die ein Träger fester, negativer Ladungen ist und ein neutrales oder kationisches Polymer enthält, vor der Sterilisation die eine oder andere der folgenden elektrischen Eigenschaften aufweist :

  - der elektrokinetische Index "I" der Membran, die ein neutrales oder kationisches Polymer enthält, ist niedriger oder gleich 0,8, wobei "I" dem Logarithmus der Proportion $|Z|$/R zur Basis 10 entspricht, und "Z" der Betrag des Potentials, ausgedrückt in Mikrovolt, der Oberfläche der Membran ist, die in Berührung mit dem Blut oder Plasma kommen soll, und "R" die elektrische Resistivität der Membran in Ohm-Zentimeter darstellt

- wobei das Zeta-Potential "Z" positiv ist und zwischen 0 und + 15 mV, Grenzwerte nicht eingeschlossen, liegt.

**Claims**

1. Use of a neutral or cationic polymer for preventing the activation of blood or plasma contact phase coming into contact with a semipermeable polyacrylonitrile-based membrane carrying fixed negative charges, provided in an apparatus for the treatment of blood or plasma by extracorporeal circulation, wherein:

   - the neutral or cationic polymer is combined with this semipermeable membrane before or after formation of the membrane and before sterilisation of the membrane;

   - total ionic strength of the semipermeable polyacrylonitrile-based membrane carrying fixed negative charges and containing a neutral or cationic polymer is less than -30 µeq/g of blown polymer;

   - total ionic strength of the semipermeable polyacrylonitrile-based membrane carrying fixed negative charges and containing a neutral or cationic polymer is, as an absolute value, at most 10% less than the total ionic strength of the semipermeable membrane without neutral or cationic polymer;

   - the semipermeable polyacrylonitrile-based membrane carrying fixed negative charges and containing a neutral or cationic polymer confirms before sterilisation one or other of the two following electric characteristics:

     • electrokinetic index 'I' is lower than or equal to 0.8, '1' corresponding to the common logarithm of the |Z|/R ratio, wherein 'Z' is the zeta potential expressed in microvolt, as an absolute value, of the surface of the membrane intended to come into contact with blood or plasma, and wherein 'R' is the electric resistivity of the membrane expressed in ohm centimetre,
     • or, the zeta potential 'Z' is positive and comprised between 0 and +15 mV, not including limits.

2. Use according to claim 1, **characterised in that** the electrokinetic index 'I' of the semipermeable polyacrylonitrile-based membrane carrying fixed negative charges and containing a neutral or cationic polymer is at most equal to or less than 0.7.

3. Use according to claim 2, **characterised in that** the electrokinetic index 'I' of the semipermeable polyacrylonitrile-based membrane carrying fixed negative charges and containing a neutral or cationic polymer is at most equal to or less than 0.6.

4. Use according to claim 1, **characterised in that** the zeta potential 'Z' is positive, is greater than or equal to +1 mV and strictly less than +15 mV.

5. Use according to claim 1, **characterised in that** the zeta potential 'Z' is positive, is greater than or equal to +6 mV and strictly less than +15 mV.

6. Use according to claims 1 to 5, **characterised in that** the total ionic strength of the semipermeable polyacrylonitrile-based membrane carrying fixed negative charges and containing a neutral or cationic polymer is less than -50 µeq/g of blown polymer.

7. Use according to any preceding claim, **characterised in that** the total ionic strength of the semipermeable polyacrylonitrile-based membrane carrying fixed negative charges and containing a neutral or cationic polymer is, as an absolute value, at most 1% less than the total ionic strength of the semipermeable membrane without neutral or cationic polymer.

8. Use according to any preceding claim, **characterised in that** the polymer is neutral and has an average molecular mass that is greater than 40.000 Daltons.

9.  Use according to claim 8, **characterised in that** the neutral polymer has an average molecular mass that is greater than 100.000 Daltons.

10. Use according to claim 8 or 9, **characterised in that** the neutral polymer is chosen from the group constituted by polyvinylpyrrolidones and polyethyleneglycols.

11. Use according to any claim 1 to 7, **characterised in that** the polymer is cationic and has an average molecular mass that is greater than 25.000 Daltons.

12. Use according to claim 11, **characterised in that** the cationic polymer has an average molecular mass that is greater than 100.000 Daltons.

13. Use according to claim 11, **characterised in that** the cationic polymer is chosen from polyamines, preferably polyethyleneimines.

14. Use according to claim 11, **characterised in that** the cationic polymer is chosen from dimethylaminoethyl dextran.

15. Use according to any preceding claim, **characterised in that** the polyacrylonitrile constituting the semipermeable membrane is a copolymer of the acrylonitrile and at least one anionic or anionizable monomer containing possibly units originating from at least one other non-saturated olefin monomer capable of being copolymerized with acrylonitrile.

16. Use according to claim 15, **characterised in that** the polyacrylonitrile is a copolymer of the acrylonitrile and of one anionic or anionizable non-saturated olefin comonomer, carrying anionic groups chosen from the sulphonate, carboxyl, phosphate, phosphonate and sulphate groups.

17. Use according to claim 16, **characterised in that** the comonomer is sodium methallyl sulphonate.

18. Use according to claims 1 to 14, **characterised in that** the polyacrylonitrile constituting the semipermeable membrane is a copolymer of the acrylonitrile and of at least one anionic or anionizable monomer containing possibly units originating from at least one other non-saturated olefin monomer capable of being copolymerized with the acrylonitrile.

19. Manufacturing process for an apparatus for the treatment of blood or plasma by extracorporeal circulation for preventing the activation of the contact phase and comprising a semipermeable membrane in the form of a planar membrane or a bundle of hollow fibres, polyacrylonitrile-based, carrying fixed negative charges, **characterised in that** it comprises the following phases:

- preparing a solution consisting of:

    - at least one polyacrylonitrile carrying fixed negative charges,
    - at least one neutral polymer,
    - at least one solvent of said polyacrylonitrile and of neutral polymer,
    - possibly at least one non-solvent of polyacrylonitrile,

- extruding this solution to form a hollow fibre or a planar membrane;
- simultaneously in the case of preparation of a hollow fibre or after extrusion in the case of formation of a planar membrane, solidifying the membrane obtained by a phase inversion process by partial or total contact of the extruded product with a liquid or gaseous and chemically alert fluid in relation to said polymers;
- washing the planar membrane or hollow fibre obtained;
- if necessary glycerising the planar membrane or hollow fibre;
- preparing a semipermeable membrane from the planar membrane or forming a bundle of hollow fibres from the hollow fibre;
- fitting the planar membrane or the bundle of hollow fibres in the casing and possibly fixing the connectors to the casing;
- sterilising the medical apparatus thereby obtained,

and adjusting the quantity of neutral polymer in order that:

- total ionic strength of the semipermeable polyacrylonitrile-based membrane carrying fixed negative charges and containing a neutral polymer is less than -30 μeq/g of blown polymer;
- total ionic strength of the semipermeable polyacrylonitrile-based membrane carrying fixed negative charges and containing a neutral polymer is, as an absolute value, at most 10% less than total ionic strength of the semipermeable membrane without neutral polymer;
- the semipermeable polyacrylonitrile-based membrane carrying fixed negative charges and containing a neutral polymer confirms before sterilisation one or other of the following electric properties:

  - electrokinetic index 'I' of the semipermeable membrane containing a neutral polymer, which is equal to $Log_{10}(|Z|/R)$ i.e. to the common logarithm of the ratio $|Z|/R$ of zeta potential 'Z', as an absolute value, expressed in microvolt, of the surface of the membrane intended to come into contact with the blood or plasma in relation to the electric resistivity 'R' of the membrane expressed in ohm. centimetre, is lower than or equal to 0.8
  - wherein the zeta potential 'Z' is positive and varies between 0 and +15 mV, not including limits.

**20.** Manufacturing process for an apparatus for the treatment of blood or plasma by extracorporeal circulation for preventing the activation of the contact phase and comprising a semipermeable membrane in the form of a planar membrane or a bundle of hollow fibres, polyacrylonitrile-based, carrying fixed negative charges, **characterised in that** it comprises the following phases:

> (a) preparing a planar membrane or hollow fibre, that is if necessary glycerised, from a polyacrylonitrile solution carrying negative charges;
> (b) assembling the different components of the apparatus, in particular fitting the semipermeable membrane or a bundle of hollow fibres in a casing and making the connectors of the casing;
> (c) simultaneously or subsequently deglycerising if necessary the semipermeable membrane and preparing a solution containing the cationic polymer in the dissolved state and placing this solution in contact with the surface of the semipermeable membrane intended to be placed in contact with the blood, it being possible to carry out phase (c) before or after phase (b);
> (d) if said phase (c) is carried out after phase (b), drain the solution containing the cationic polymer from the apparatus;
> (e) if necessary rinsing the semipermeable membrane to eliminate excess non-fixed cationic polymer and if necessary glycerising the semipermeable membrane again;
> (f) sterilising the medical apparatus;

and adjusting the quantity of cationic polymer in order that:

- total ionic strength of the semipermeable polyacrylonitrile-based membrane carrying fixed negative charges and containing a cationic polymer is less than - 30 μeq/g of blown polymer;

- total ionic strength of the semipermeable polyacrylonitrile-based membrane carrying fixed negative charges and containing a cationic polymer is, as an absolute value, at most 10% less than total ionic strength of the semipermeable membrane without cationic polymer;

- the semipermeable polyacrylonitrile-based membrane carrying fixed negative charges and containing a cationic polymer confirms before sterilisation one or other of the following electric properties:

  - electrokinetic index 'I' of the semipermeable membrane containing a cationic polymer, which is equal to $Log_{10}(|Z|/R)$ i.e. to the common logarithm of the ratio $|Z|/R$ of zeta potential 'Z', as an absolute value, expressed in microvolt, of the surface of the membrane intended to come into contact with the blood or plasma in relation to the electric resistivity 'R' of the membrane expressed in ohm.centimetre, is lower than or equal to 0.8,
  - or the zeta potential 'Z' is positive and varies between 0 and +15 mV, not including limits.

**21.** Manufacturing process for an apparatus for the treatment of blood or plasma by extracorporeal circulation for preventing the activation of the contact phase and comprising a semipermeable membrane in the form of a planar membrane, polyacrylonitrile-based, carrying fixed negative charges, **characterised in that** it comprises the following phases:

(a) preparing a planar membrane, that is if necessary glycerised, from a polyacrylonitrile solution carrying negative charges;

(b) simultaneously or subsequently deglycerising if necessary the semipermeable membrane and preparing a solution containing the cationic or neutral polymer in the dissolved state and pulverising this solution on the surface of the semipermeable membrane intended to be placed in contact with the blood;

(c) assembling the different apparatus components, in particular fitting the semipermeable membrane in a casing and making the connectors of this casing;

(d) if necessary rinsing the semipermeable membrane to eliminate excess non-fixed cationic polymer and if necessary glycerising the semipermeable membrane again;

(e) sterilising the medical apparatus;

and adjusting the quantity of cationic or neutral polymer in order that:

- total ionic strength of the semipermeable polyacrylonitrile-based membrane carrying fixed negative charges and containing a cationic or neutral polymer is less than 30 μeq/g of blown polymer;

- total ionic strength of the semipermeable polyacrylonitrile-based membrane carrying fixed negative charges and containing a cationic or neutral polymer is, as an absolute value, at most 10% less than total ionic strength of the semipermeable membrane without cationic or neutral polymer;

- the semipermeable polyacrylonitrile-based membrane carrying fixed negative charges and containing a cationic or neutral polymer confirms before sterilisation one or other of the following electric properties:

  - electrokinetic index 'I' of the semipermeable membrane containing a neutral or cationic polymer, which is equal to $Log_{10}(|Z|/R)$ i.e. to the common logarithm of the ratio $|Z|/R$ of zeta potential 'Z', as an absolute value, expressed in microvolt, of the surface of the membrane intended to come into contact with the blood or plasma in relation to the electric resistivity 'R' of the membrane expressed in ohm.centimetre, is lower than or equal to 0.8,
  - wherein the zeta potential 'Z' is positive and varies between 0 and +15 mV, not including limits.

22. Apparatus for the treatment of blood or plasma by extracorporeal circulation that is sterilised and ready for use, for preventing the activation of the contact phase and comprising a semipermeable membrane in the form of a planar membrane or a bundle of hollow fibres, polyacrylonitrile-based, carrying fixed negative charges, **characterised in that** before or after formation of the semipermeable membrane and before sterilisation, at least one neutral or cationic polymer is incorporated into the semipermeable membrane in an appropriate quantity in such a way that:

- total ionic strength of the semipermeable polyacrylonitrile-based membrane carrying fixed negative charges and containing a neutral or cationic polymer is less than -30 μeq/g of blown polymer;

- total ionic strength of the semipermeable polyacrylonitrile-based membrane carrying fixed negative charges and containing a neutral or cationic polymer is, as an absolute value, at most 10% less than total ionic strength of the semipermeable membrane without neutral or cationic polymer;

- the semipermeable polyacrylonitrile-based membrane carrying fixed negative charges and containing a neutral or cationic polymer confirms before sterilisation one or other of the following electric properties:

  - electrokinetic index 'I' of the membrane containing a neutral or cationic polymer is lower than or equal to 0.8, 'I' being equal to the common logarithm of the ratio $|Z|/R$ wherein 'Z' is zeta potential, as an absolute value, expressed in microvolt, of the surface of the membrane intended to come into contact with the blood or plasma and wherein 'R' is the electric resistivity of the membrane expressed in ohm.centimetre,
  - wherein the zeta potential 'Z' is positive and varies between 0 and +15 mV, not including limits.